(19) **Europäisches Patentamt / European Patent Office / Office européen des brevets**

(11) **EP 2 589 390 B1**

(12) **EUROPEAN PATENT SPECIFICATION**

(45) Date of publication and mention of the grant of the patent:
**07.03.2018 Bulletin 2018/10**

(21) Application number: **11801210.3**

(22) Date of filing: **09.06.2011**

(51) Int Cl.:
*A61K 35/30* (2015.01)          *A61P 25/00* (2006.01)
*A61K 38/17* (2006.01)

(86) International application number:
**PCT/RU2011/000406**

(87) International publication number:
**WO 2012/002840 (05.01.2012 Gazette 2012/01)**

(54) **PHARMACEUTICAL COMPOSITION FOR TREATING CENTRAL AND PERIPHERAL NERVOUS SYSTEM DISEASES OF VASCULAR, TRAUMATIC, TOXIC, HYPOXIC AND AUTOIMMUNE ORIGIN**

PHARMAZEUTISCHE ZUSAMMENSETZUNG ZUR BEHANDLUNG VON ERKRANKUNGEN DES ZENTRALEN UND PERIPHEREN NERVENSYSTEMS VASKULÄREN, TRAUMATISCHEN, TOXISCHEN, HYPOXISCHEN UND AUTOIMMUNEN URSPRUNGS

COMPOSITION PHARMACEUTIQUE DESTINÉE AU TRAITEMENT DES AFFECTIONS DU SYSTÈME NERVEUX CENTRAL ET DU SYSTÈME NERVEUX PÉRIPHÉRIQUE AYANT UNE GENÈSE VASCULAIRE, TRAUMATIQUE, TOXIQUE, HYPOXIQUES OU AUTO-IMMUNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.07.2010 RU 2010126868**

(43) Date of publication of application:
**08.05.2013 Bulletin 2013/19**

(73) Proprietor: **Aktsionernoe Obschestvo "Pharm-Sintez"**
**Moskow, 111024 (RU)**

(72) Inventors:
• **NAZARENKO, Anna Borisovna**
  **Moscow 121614 (RU)**
• **SOKOLOV, Mikhail Anatolevich**
  **Moscow 107150 (RU)**

(74) Representative: **Held, Stephan et al**
**Meissner Bolte Patentanwälte**
**Rechtsanwälte Partnerschaft mbB**
**Widenmayerstraße 47**
**80538 München (DE)**

(56) References cited:
**EP-A1- 0 249 563          WO-A1-2009/088314**
**WO-A1-2010/007620     WO-A1-2010/007620**
**WO-A2-02/064748        EA-B1- 003 301**
**RU-C1- 2 104 702**

• **A. H. Fischer ET AL: "Cryosectioning Tissues", Cold Spring Harbor Protocols, vol. 2008, no. 9, 1 August 2008 (2008-08-01), pages pdb.prot4991-pdb.prot49, XP055315924, DOI: 10.1101/pdb.prot4991**

## Description

PERTINENT ART

The invention relates to the medicine, in particular, to the pharmacology, pharmaceutical industry, and veterinary medicine; it is the pharmaceutical composition with reparative-regenerative effect on the nervous tissue, intended to treat the vascular, traumatic, toxic, hypoxic, and autoimmune genesis central and peripheral nervous system diseases, which consists of the biologically active protein-polypeptide complex that includes the organo-specific nervous tissue growth and differentiation factors, and signaling molecules in therapeutically efficient ratios and concentrations, and a pharmaceutically acceptable diluent. The pharmaceutical composition is a biologically active protein-polypeptide complex, wherein the molecular masses of the forming it protein-polypeptide components are within the 5 to 200 kDa range; the 10 to 120 kDa range medium molecular faction makes min. 80%; the total protein concentration is within the 0.8 to 4.2 mg/ml range; the maximum solution UV spectrum absorption is at the $280\pm5$ nm wave length; with a peak in the UV-visible spectrum at the 274-284 nm wave length; with bands in the pI 4.2 to 8.4 range at isoelectric focusing in a 5% polyacrylamide gel; comprising a pharmaceutically acceptable diluent comprising a buffer solution and excipients: high-molecular compounds, stabilizers, preservatives, and solubilizers, all in sufficient concentrations. The substance has been obtained by the ion exchange chromatography of the mid first third to mid last third hoof livestock embryonic brain tissue homogenate filtrate, which chromatography is performed in presence of the buffer solution, detergents, and proteolysis inhibitors.

PRIOR ART

A number of the animal feedstock nootropic and neurometabolic activity protein-peptide nature drugs is known, which drugs are used to treat the nervous system diseases. The closest as of the achievable effect ones are:

- Cerebrolysin: drug to treat the hemorrhagic or ischemic strokes, by Ebewe (Austria) [1], a product of intact pigs brain treatment;
- Cerebrocurin: drug for diseases associated with the central nervous system dysfunctions, by OOO "NIR" (Ukraine) [2], a product of animal embryos brain treatment;
- Cortexin: polypeptide nature drug obtained by extraction from the bovine and porcine brain cortex, by OOO "Geropharm" (Russia) [3], with the nootropic, cerebroprotective, anticonvulsant, and antioxidative effects;
- Cerebrolysate: drug improving the resistance of the brain tissue to the intoxication, hypoxia, hypoglycemia, and mechanical injury, by "Microgen" scientific-production association, a federal state unitary enterprise (Immunopreparat) [4], with the nootropic effect, a hydrolysate of the bovine cattle brain cortex.

The Cerebrolysin includes the amino acids (~85%), low molecular weight peptides (-15%), and microelements, the feedstock for which is the porcine brain. The neuroprotective and trophic effects of the drug are due to the specific peptides and amino acids, of max. 10,000 Dalton molecular mass, where the alanine, leucine, and lysine dominate and determine the drug properties. The drug is intended for the intramuscular and intravenous administration, the biologically active components concentration is low; hence, the treatment is high dose and long term. The detriments of the known drug are the long term treatment, low activity and specificity due to the feeble neuroprotective effect; and extremely low regenerative and reparative potentials for the nervous tissue. The Cerebrocurin contains the water-soluble prenatal neuropeptides and the products of cleavage of the inactive high molecular weight precursors of the animal embryos brain homogenate, this homogenate is, after dilution in physiological solution, exposed to the immobilized proteolytic enzyme; the interaction conditions are defined on the basis of the target preparation check sample; and the resulting solution undergoes a 30-day cure at max. 10°C. This technique offers a higher (compared with the Cerebrolysin) activity of the resulting drug and significantly expands its spectrum of action, due to the higher mass of the peptides and low molecular weight proteins remaining after proteolysis. However, despite the long time solution formation before the aggregation and proteolysis processes end, this technique does not yield the purity required for the intravenous use; hence, the lesser bioavailability. The aqueous extraction without the solution, detergents, proteolysis inhibitors, and solubilizers buffering at the Cerebrocurin production phases, and the undetermined animal brain gestation terms do not allow for the necessary standardization; hence, the significant discrepancies as of the evolutionarily fixed protein-peptide concentrations ratios, and lower tissue-specific neuroregenerative activity and pharmacologic effect stability pharmacologic, all being limited to the feedback type biologic effect due to the tissue proteolysis products signal function.

The Cortexin contains the complex of the water-soluble biologically active alkaline, acid, and neutral polypeptides with the 500 to 15000 Da molecular mass and 3.5 to 9.5 isoelectric point. These are obtained from the livestock brain blenderized frozen tissue; the process consists in extracting with the zinc chloride containing acetic acid solution, separating the sediment, treating the supernatant liquid with acetone, drying with further purifying, sterilizing, and freeze drying the target product.

The drug is intended for the intramuscular and intravenous administration, the biologically active low molecular weight components concentration is low; hence, the treatment is high dose and long term. Though the Cortexin participates in regulating the inhibitory and exciting amino acids ratios, and concentrating the serotonin and dopamine, has a GABA-positive effect, reduces the toxic effects of the neurotropic drugs, improves the learning and memory processes, accelerates the brain functions rehabilitation after stresses, its activity and specificity are not high due to the feeble neuroprotective effect, its regenerative potential is low and reparative potential is insignificant for the nervous tissue; hence, the long term protracted treatment.

The Cerebrolysate also contains the water-soluble polypeptides obtained in the bovine brain cortex hydrolysis; its enzymatic activity is feeble; its molecular mass is max. 15000 Da. The mentioned in the Patent variation of the drug amino acid composition admits a significant compositional variation of the resulting peptide concentration ratios, determining the low tissue-specific activity and pharmacologic effect stability with the feeble neurometabolic and unexpressed nootropic effects.

The nervous system vascular diseases and traumatic, toxic, and hypoxic brain and spinal cord injuries incidence growth (road accidents, manmade and natural disasters, etc.) incites the pharmacologist to search for the new, more efficient The nervous system vascular diseases and traumatic, toxic, and hypoxic brain and spinal cord injuries incidence growth (road accidents, manmade and natural disasters, etc.) incites the pharmacologist to search for the new, more efficient drugs to treat with success the neurological and neurosurgical patients, to rehabilitate them, and to reduce the population disability level.

WO 2010/007620 A1 discloses pharmaceutical compositions comprising an extract of fetal porcine brain tissue and the use thereof in the treatment of diseases.

WO 2009/088314 A1 relates to a method for producing a biologically active complex having neuroregenerative action.

DISCLOSURE OF INVENTION

The purpose of the Invention was to create the pharmaceutical composition with an expressed reparative-regenerative effect on the nervous tissue, to treat the vascular, traumatic, toxic, hypoxic, and autoimmune genesis central and peripheral nervous system diseases.

The desired objective is reached by that the pharmaceutical composition for use in the treatment of vascular, traumatic, toxic, hypoxic, and autoimmune genesis central and peripheral nervous system diseases has the form of the solution for parenteral, intranasal, and subconjunctival administration; and its active ingredient is the 0.01 to 2.0 mg/ml biologically active protein-polypeptide complex with the tissue-specific reparative effect on the nervous tissue, obtained from the mid first third to mid last third gestational age livestock quickly frozen embryonic brain tissue, which complex contains the negatively charged faintly acid neutral proteins and polypeptides relating to the growth factor, differentiation factor, signaling molecules, that determine its biologic and pharmacologic activity, with the 5 to 200 kDa molecular masses, wherein at least 80% of the total protein mass has the 10 to 120 kDa molecular mass, and characterized by a peak at the 274-284 nm wave length in the UV-visible spectrum and bands in the pI 4.2 to 8.4 range at isoelectric focusing in a 5% polyacrylamide gel, and which complex contains a pharmaceutically acceptable diluent.

With all this, the preferable pharmaceutical composition diluent is an officinal buffer solution with excipients, including the high molecular weight compounds, stabilizers, preservatives, and solubilizers.

Thus, the desired objective is reached by the described pharmaceutical composition wherein the active ingredient is the biologically active protein-polypeptide complex consisting of the 5 to 200 kDa molecular mass negatively charged faintly acid neutral proteins and polypeptides, obtained from the mid first third to mid last third gestational age livestock quickly frozen embryonic brain tissue, which complex contains the organo-specific nervous tissue growth and differentiation factors, and signaling molecules, in therapeutically efficient ratios and

molecular weight compounds, e.g., carmellose; and it contains the stabilizers, e.g., mannitol, preservatives, e.g., Nipagin, and solubilizers, e.g., Polysorbate 20. The claimed pharmacologic composition, which consists of the protein-polypeptide complex and pharmaceutically acceptable diluent differs from the earlier obtained substances and compositions extracted from the animal brain, as of the feedstock source at the fixed gestational ages, as of the molecular masses (max. 200 kDa) and qualitative composition of the protein-polypeptide fractions, as of the evolutionarily fixed in ontogenesis growth proteins, differentiation factors, and signaling molecules concentration ratios, as of the method of administration (parenteral, intrathecal, intranasal, subconjunctival), as of the safety of use (no toxicity, mutagenicity, carcinogenicity, and/or allergenicity), and as of the biological and pharmacological activity and this activity specificity (tissue- and organo-specific, reparative-regenerative).

BEST EMBODIMENT OF INVENTION

**Example 1. Biologically active complex production method.**

Protein fraction production from porcine embryonic brain. 250 grams of quick-frozen porcine embryonic brain, 14 to 18 weeks gestational age, is unfrozen and 7-minute homogenized in 1200 ml of 0.05 M TRIS-glycin-phosphate buffer, pH 7.0, containing 1 mM EDTA and 0.1% maltose, at 8°C. The homogenate is filtered through a close cloth to separate the ballast substances, then it is 30-minute centrifuged at 18,000 g, and then filtered through a 0.45 $\mu$m mesh membrane filter at 8°C. The filtrate is then applied to a 200 ml volume chromatographic column with the Toyopearl DEAE-650M anion-exchange medium; the column is equilibrated with 4 volumes of the TRIS-glycin-phosphate buffer, pH 7.0, containing 0.1 mM EDTA and 0.1 mM NaCl. The proteins linked to the carrier are separated by the stepwise gradient of the 105-step increasing the salt concentration, and collecting the target fractions. Chromatography temperature: 8°C. The target fraction is stage by stage ultra filtered at max. 8.0 kfg/cm$^2$ back pressure, through 5 kDa and 200 kDa retention potential materials; then it is desalinized, and diluted with the 0.05 M glycin-NaOH solution, pH 7.4, down to the 1.0 mg/ml protein concentration. The solution is sterilizing filtered, through a max. 0.22 $\mu$m mesh membrane filter. To characterize the resulting protein fraction the ultraviolet spectrophotometry, gel chromatography, electrophoresis in polyacrylamide gel, and amino acid analysis were used. The solution ultraviolet spectrum is read in the 250 to 350 nm wave length range, the maximum absorption is observed at 280±5 nm. To determine the molecular mass of the complex forming proteins and polypeptides the following methods are used: gel chromatography with Superdex 75 (GE Healthcare) and denaturing SDS-Page electrophoresis in 12-percent polyacrylamide gel in comparison with a standard marker proteins set. The column is calibrated with the protein standards, molecular masses range: 13 kDa to 200 kDa. The above methods have found that the drug contains the negatively charged faintly acid neutral proteins and polypeptides relating to the growth factor, differentiation factor, 5 to 200 kDa molecular mass signaling molecules, wherein at least 82% of the total protein mass has the 10 to 120 kDa molecular mass, and wherein a peak exists at the 274-284 nm wave length in the UV visible spectrum and bands in the pI 4.2 to 8.4 range at isoelectric focusing in a 5% polyacrylamide gel; and which drug contains a pharmaceutically acceptable diluent.
Amino acid analysis (%): Asp: 10.82 ± 2.8; Thr: 5.4 ± 1.6; Ser: 5.2 ± 1.8;
Glu: 16.2 ± 3.4; Pro: 7.045 ± 2.5; Gly: 5.2 ± 2.4; Ala: 5.4 ± 1.8; Val: 7.08 ± 2.9; Met: 2.65 ± 1.4; He - 4.45 ± 1.8; Leu: 9.4 ± 2.6; Tyr: 4.02 ± 1.2; Phe: 4.8 ± 1.9; Orn: 0.48 ± 0.2; Lys: 8.48 ± 2.4; His: 2.8 ± 0.9; and Arg: 6.52 ± 2.2.

**Example 2. Biologically active complex production method.**

Protein fraction production from the porcine embryonic brain. 200 grams of quick-frozen porcine embryonic brain, 3 to 4 weeks gestational age, is unfrozen and 5-minute homogenized in 800 ml of 50 mM TRIS-maleate buffer, pH 5.8, containing 1 mM EDTA and 0.1% mannitol, at 25°C. The homogenate is filtered through a close cloth to separate the ballast substances, then it is 30-minute centrifuged at 30,000 g, and then filtered through a 0.45 $\mu$m mesh membrane filter at 25°C. The filtrate is then applied to a 250 ml volume chromatographic column with the DEAE-Sepharose anion-exchange medium; the column is equilibrated with 4 volumes of the maleate buffer solution, pH 5.8, containing 0.1 mM EDTA. The proteins linked to the carrier are separated by the stepwise gradient of the maleate buffer solution, pH 5.8, containing 0.04 M NaCl, 0.02 M-step increasing the salt concentration; the target fractions collection starts at the 0.06 M salt concentration. Chromatography temperature: 2 to 4°C. The target fraction is stage by stage ultra filtered at max. 8.0 kfg/cm$^2$ back pressure, through 5 kDa and 200 kDa retention potential materials; then desalinized, and diluted with the 50 M aspartate-NaOH solution, pH 7.4, down to the 1.0 mg/ml protein concentration, adding the polyoxyethylenesorbite monooleate (Tween 20) to the 0.01 mg/ml total concentration. The solution is sterilizing filtered, through a max. 0.22 $\mu$m mesh membrane filter. To characterize the resulting protein-polypeptide fraction the ultraviolet spectrophotometry, gel chromatography, electrophoresis in polyacrylamide gel, and amino acid analysis were used. The solution ultraviolet spectrum is read in the 250 to 350 nm wave length range, the maximum absorption is observed at 280±5 nm. To determine the molecular mass of the complex forming proteins and polypeptides the following methods are used: gel chromatography with Superdex 75 (GE Healthcare) and denaturing SDS-Page electrophoresis in 12-percent polyacrylamide gel in comparison with a standard marker proteins set. The column is calibrated with the protein standards, molecular masses range: 13 kDa to 250 kDa. The above methods have found that the drug contains the negatively charged faintly acid neutral proteins and polypeptides relating to the growth factor, differentiation factor, 5 to 200 kDa molecular mass signaling molecules, wherein min. 82% of the total protein mass has the 10 to 120 kDa molecular mass, and wherein a peak exists at the 284 nm wave length in the UV visible spectrum and bands in the pI 4.2 to 8.4 range at isoelectric focusing in a 5% polyacrylamide gel; and which drug contains a pharmaceutically acceptable diluent.
Resulting complex amino acid analysis (%): Asp: 10.82 ± 1.3; Thr: 5.4 ± 0.9; Ser: 5.2 ± 1.1; Glu: 16.2 ± 1.9; Pro: 7.045 ± 1.7; Gly: 5.2 ± 0.8; Ala: 5.4 ± 1.1; Val: 7.08 ± 2.3; Met: 2.65 ± 0.6; He: 4.45 ± 0.8; Leu: 9.4 ± 2.5; Tyr: 4.02 ± 0.6; Phe: 4.8 ± 1.1; Orn: 0.48 ± 0.1; Lys: 8.48 ± 2.3; His: 2.8 ± 0.7; Arg: 6.52 ± 2.1.

**Example 3. Pharmaceutical composition production method.**

The obtained as of Example 1 porcine embryonic brain biologically active complex solution is diluted with the 50 mM glycin-phosphate buffer solution, containing, in total, 0.5% mannitol, 0.1% carmellose, 0.0005% Polysorbate 80 and disubstituted sodium phosphate to pH 8.0 to get the final total protein in resulting solution concentration within the 0.9 to 1.2 mg/ml range. I.e., the composition may contain the 0.9, 1, and/or 1.2 mg/ml biologically active protein-polypeptide complex.

The resulting pharmaceutical composition is sterilizing filtered, through a max. 0.1 $\mu$m mesh membrane filter, dispensed in glass vials, and plugged. The resulting pharmaceutical composition properties have been assessed following the conventional experimental (preclinical) study of new pharmacological substances guidelines [5].

**Example 4. Pharmaceutical composition production method.**

The obtained as of Example 2 porcine embryonic brain biologically active complex solution is diluted with the 50 mM maleate buffer solution, pH 5.8, containing, in total, 0.3% lactic acid, 0.5% mannitol, 0.1% carmellose, up to 0.0005% Polysorbate 80 to get the final total protein in resulting solution concentration within the 0.9 to 1.2 mg/ml range, i.e., the pharmaceutical composition may contain 0.9, 1, and/or 1.2 mg/ml biologically active protein-polypeptide complex. The resulting pharmaceutical composition is sterilizing filtered, through a max. 0.1 $\mu$m mesh membrane filter, dispensed in glass vials, and plugged.

**Example 5. Toxicity study**

The toxicity was tested in the acute and subchronic experiments with the original pharmaceutical composition. Pharmaceutical form: sterile intranasal dosing spray, in 10 and 30 ml glass (0.1 mg/ml) glass or polymer vial; solution for injection: 5 ampoules 1 ml each (0.1 mg/ml), 5 ampoules 2 ml each (0.1 mg/ml), and 1 ampoule 2 ml (0.2 mg/ml). Maximum recommended daily doses: 0.4 mg endolumbarly, and 0.1 mg intravenously.

*Experimental conditions and methods*

The study purpose was to determine the original pharmaceutical composition tolerable, toxic, and lethal doses. The experiment used the both sexes mice, BALB/C line, 18 to 20 g body mass, intragastrically and intraperitoneally. Observation term: 14 days. The animals were received from nursery, certified. The animals were kept in standard condition, plastic cages, 10 animals each, 10 animals in group. Feed: combined feed, fresh vegetables, curds. Free access to water and feed. Vivarium light: artificial. Air temperature: 18 to 20°C. The maximum possible for administration to mice dose was 0.2 ml, or 10 mg/kg, i.e., 600-fold daily therapeutic dose. The animals were withdrawn from experiment with ether narcosis; the animals were prosected; and the viscera were analyzed.

*Acute toxicity study results.*

*Mice.* Body mass: 18 to 20 grams. Administered dose: 0.2 ml, or 10 mg/kg.
Animals in group: 10. Two groups: intragastrical and intraperitoneal. Observation
term: 14 days.
*Habitus.* To the end of experiment all the animals looked healthy. Hair: dense, white color. No differences between groups as of habitus.
*Behavior.* No differences between groups, normal.
*Death rate.* No fatalities in any of the three groups.
*Body mass.* Six control weighings during experiment. No loss or growth revealed. *Viscera.* No differences between groups as of viscera conditions. No viscera alterations or augmentation.

**Subacute (subchronic) toxicity study**

*Experimental conditions and methods*

The original pharmaceutical composition subchronic toxicity experimental study used the 200 to 250 grams body weight Wistar male and female rats. The animals were received from nursery. The animals were kept in standard condition, plastic cages, 5 animals each. Feed: cubed feed, fresh vegetables, curds. Free access to water and feed.

Vivarium light: artificial. Air temperature: 18 to 200°C. Before experiment the animals were 10 days kept in quarantine. Ten animals (5 males and 5 females) in group. Groups segregation:

Group 1:     intact animals (control);
Group 2:     original pharmaceutical composition, therapeutic dose, subcutaneous;
Group 3:     original pharmaceutical composition x 10, subcutaneous;
Group 4:     original pharmaceutical composition, intravenous, therapeutic dose; and
Group 5:     original pharmaceutical composition x 10, intravenous.

Observation term: 1 month administration, 1 month resorptive effect observation. Toxicity assessment according to observation check list points:

> survival rate and habitus: (hair, eyes, ears, extremities, teeth);
> status and behavior (activity, walking, temperament, eating);
> body mass change (weighing before and at the end of the experiment); and
> physiologic functions (respiration, salivation, urination, excreta).

Before and at the end of the experiment the animals were blood tested: peripheral blood morphology (erythrocytes, leucocytes, thrombocytes counts, hemoglobin level) and biochemistry (albumin, urea, creatinine, glucose levels); activity of certain enzymes (aspartate aminotransferase, alanine aminotransferase, alkaline phosphatase, lactate dehydrogenase). The blood for hematologic study was taken from the tail vein. To count the blood corpuscles the Picoscale (Hungary) automatic counter was used. The hemoglobin level was determined by the cyanmethemoglobin method. To determine the biochemistry parameters the FP-901, Labsystems, Finland, automated system was used. After the chronic experiment completion the animals were sacrificed by narcosis overdose, for pathomorphological exams of the organs and tissues. The animals were prosected right after death, complete pathological study pattern. For the pathomorphological exams, the tissue samples were gelatinized and cryostatted to obtain the up to 5 $\mu$m thick slices. Then the tissues were fixed and hematoxylin-eosin stained. For the microscopy the Opton (Germany) microscope was used. The experimental data were compared with the control ones and counted using a statistical software.

## *Results of Study. Macroscopic.*

> *Survival rate*: All the experimental animals tolerated all the administration methods. No deaths were observed in either the experimental or control groups.
> *Behavior*: No behavior (hyperactivity or hypoactivity) or status deviations of the experimental animals compared with the control ones were recorded. Muscular tone hypererethism: none.
> *Habitus.* all animals were of medium finish; no atrophies or obesities. Hair: smooth and bright; no falloffs or fragility. Corneal haze, lacrimation, other pathologies: none. Earflaps: rose color, no scabs, no inflammations, no jerks. Teeth: regular color in all animals, no breaks.
> *Functions:* respiration: quiet, regular rhythm, unobstructed in both experimental and control; salivation: no pathologies; urination: rate, volume, and color within the physiologic norm.
> *Clinical:* Body mass dynamics: positive in all experimental groups. No firm differences between experimental and control.
> *Hematology*: hemoglobin level; erythrocytes, leucocytes, and thrombocytes counts: within the physiologic norm in all groups.
> *Biochemistry:* the peptide complex test dose did not affect the blood serum protein in the experimental animals; hence, no damaging effect of the tested drug on the liver protein-forming function. To reveal an eventual adverse hepatic effect, the aspartate aminotransferase, alanine aminotransferase, and alkaline phosphatase activity in the experimental animals blood serum was determined. The data analysis has shown no reliable alterations in the said blood serum enzymes activity.
> *Pathomorphology: Macroscopic.*
> In all the animals in all the groups at prosection: clean skin, moderately developed subcutaneous fat layer. Viscera arrangement: regular; no free liquid in either the pleural or abdominal cavities. Tracheal and bronchial lumen: free; mucosa: clean, wet, and lustrous. The pulmonary tissue in the experimental groups has a mode intense coloration than in the control ones, without, however, edema signs.
> Myocardium on section: no lesions.
> Tongue: clean. Buccal cavity and esophagus mucosa: no defects. Stomach and intestines: no traces of irritation.

Liver: not enlarged.

The renal capsule is easily separable; the medullar and cortical substance on section are well distinguishable.

The spleen has a smooth capsule, grayish-brown color, no scrape on pulp. Seminal glands and ovaries: no peculiarities.

Thymus: grayish color, no hemorrhages.

Thyroid gland: dense, with symmetric lobes.

Brain tunics: moderate blood filling, wet, lustrous. Brain matter: symmetric pattern on section.

The body mass indexes of the experimental animals had no valid differences with those of the control groups.

Microscopy: In all the studied groups: no pathomorphological alterations in the viscera (liver, kidneys, lungs, heart, spleen, stomach, large and small intestines) endocrine glands (thyroid, thymus, adrenal glands, pancreatic gland), reproductive organs (uterus, ovaries), lymph nodes, brain, skin, nose and throat mucosas.

**Subchronic toxicity at intranasal administration.**

**Mature animals.**

The study used the mature female 2500 grams Chinchilla rabbits. The animals were received from nursery. The animals were kept in standard metal cages, one animal per cage. Feed: cubed feed, fresh vegetables. Free access to water and feed. Vivarium light: artificial. Air temperature: 18 to 20°C. Three group, five animals in each. Groups segregation: Group 1 (therapeutic dose): pharmaceutical composition, 0.1 mg/ml, 0.2 ml, single, daily; Group 2: pharmaceutical composition, 0.1 mg/ml, 0.2 ml, twice a day, daily; and Group 3: intact animals. Term of administration: 1 month.

Toxicity assessment according to observation check list points: survival rate, habitus, status, behavior, and body mass change (weighing before and at the end of the experiment). The local irritant effect during experiment was assessed from the nose mucosas alterations.

Before and at the end of the experiment the animals were blood tested: peripheral blood morphology (erythrocytes, leucocytes, thrombocytes counts, hemoglobin level) and biochemistry (albumin, urea, creatinine, glucose levels); activity of certain enzymes (aspartate aminotransferase, alanine aminotransferase, alkaline phosphatase, lactate dehydrogenase). The blood to determine the above was taken from the auricular veins, 1.5 to 2.0 ml.

To count the blood corpuscles the Picoscale (Hungary) automatic counter was used. The hemoglobin level was determined by the cyanmethemoglobin method. To determine the biochemistry parameters the FP-901, Labsystems, Finland, automated system was used.

After the chronic experiment completion the animals were sacrificed by narcosis overdose. The animals were prosected right after death, complete pathological study pattern.

The morphometric assessment of the animal organs parameters used the Sartorius (Germany) scale; further on, the organs' masses and their standard deviations were calculated.

For the pathomorphological exams, the fresh tissue samples were gelatinized and cryostatted to obtain the up to 5 $\mu$m thick slices. Then the tissues were fixed and hematoxylin-eosin stained. For the microscopy the Opton (Germany) microscope was used.

The experimental data were compared with the control ones and counted using a statistical software.

**Results of Study**

***1. Visual.***

*Survival rate*: All the experimental animals tolerated all the administered doses of the pharmaceutical composition. No deaths were recorded.

*Behavior*: No hyperactivity or hypoactivity in the experimental animals compared with the control ones were observed. No muscular tone hypererethism was recorded in either the experimental or control animals. It has been seen that the drug administration does not provoke discomfort in the animals.

*Habitus.* all animals, independently of the dose, were of medium finish; no atrophies or obesities. Hair: smooth and bright; no falloffs or fragility. Corneal haze, lacrimation, other pathologies: none. Earflaps: rose color, no scabs, no inflammations, no jerks. Teeth: regular color in all animals, no breaks.

*Functions:* respiration: quiet, regular rhythm, unobstructed in both experimental and control; salivation: no pathologies; urination: rate, volume, and color within the physiologic norm; and excreta: color and shape same as control.

*2. Clinical studies:*

*Body mass dynamics:* positive in all the experimental groups, no significant difference from control.

*Blood test:* hemoglobin level; erythrocytes, leucocytes, and thrombocytes counts: within the physiologic norm in all groups.

Biochemistry: blood glucose, alanine and asparagine aminotransferases activity normal in all groups. The protein concentration was not statistically different between the animals having received the pharmaceutical composition once and twice daily.

*3. Pathomorphology:*

In all the studied groups: no pathomorphological alterations in the viscera (liver, kidneys, lungs, heart, spleen, stomach, large and small intestines) endocrine glands (thyroid, thymus, adrenal glands, pancreatic gland), reproductive organs (uterus, ovaries), lymph nodes, brain, skin, nose and throat mucosas. Thus, the pharmaceutical composition nasal form subchronic toxicity has been tested. The spray subchronic test has not revealed either any significant alterations of the experimental animals viscera, or local irritant effect. The subchronic toxicity study used the clinical methods, conventional methods for the blood hematology and biochemistry, and viscera and nasal mucosas histology.

**Subchronic toxicity study in immature rabbits at intranasal administration.**

The study used the 3-week age immature 300 to 500 grams Chinchilla rabbits. The drug was administered daily, one or two times, single spray, during 2 weeks. The animals were received from nursery. The animals were kept in standard metal cages, one animal per cage. Feed: cubed feed, fresh vegetables. Free access to water and feed. Vivarium light: artificial. Air temperature: 18 to 20°C. Three group, five animals in each. Groups segregation: Group 1 (therapeutic dose): pharmaceutical composition, 0.1 mg/ml, 0.2 ml, single, daily; Group 2: pharmaceutical composition, 0.1 mg/ml, 0.2 ml, twice a day, daily; and Group 3: intact animals. Term of administration: 2 weeks.

Toxicity assessment according to observation check list points: survival rate, habitus, status, behavior, and body mass change (weighing before and at the end of the experiment). The local irritant effect during experiment was assessed from the nose mucosas alterations.

Before and at the end of the experiment the animals were blood tested: peripheral blood morphology (erythrocytes, leucocytes, thrombocytes counts, hemoglobin level) and biochemistry (albumin, urea, creatinine, glucose levels); activity of certain enzymes (aspartate aminotransferase, alanine aminotransferase, alkaline phosphatase, lactate dehydrogenase). The blood to determine the above was taken from the auricular veins, 1.5 to 2.0 ml. To count the blood corpuscles the Picoscale (Hungary) automatic counter was used. The hemoglobin level was determined by the cyanmethemoglobin method. To determine the biochemistry parameters the FP-901, Labsystems, Finland, automated system was used.

After the chronic experiment completion the animals were sacrificed by narcosis overdose. The animals were prosected right after death, complete pathological study pattern.

The morphometric assessment of the animal organs parameters used the Sartorius (Germany) scale; further on, the organs' masses and their standard deviations were calculated.

For the pathomorphological exams, the fresh tissue samples were gelatinized and cryostatted to obtain the up to 5 $\mu$m thick slices. Then the tissues were fixed and hematoxylin-eosin stained. For the microscopy the Opton (Germany) microscope was used.

The experimental data were compared with the control ones and counted using a statistical software.

**Results of Study**

***Assessment of irritant effect on nasal mucosa.***

*Intravital biomicroscopy*

During the whole observation term, the animal behavior remained normal; no ooze from the nasal passages or scabs were recorded. The drops provoked in the rabbits the sneezing reflex, stopping in 1 to 2 minutes. At the instillation of drops, both the therapeutic group and the overdose group rabbits had the mechanical irritation effect, expressed in the sneezing reflex, nose jerks; the animals rubbed their noses with their paws.

*Control intact group.* Mucosa: pale rose color, no edemas or ooze. The otolaryngologic speculum observation revealed no lesions on the epithelium. Nasal mucous secretion: transparent, hydrous. Reflexes: within the physiologic norm.

Confocal microscopy: Epithelial layer of densely packed structure of clearly differentiated cells. The cells are connected by gap junctions.

*In the therapeutic dose group,* the nasal mucosa remained clear during the whole observation term: weak rose color, no oozes or edemas. Mucosa vessels: unchanged. The epithelium state was assessed with the slit illumination. No lesions were revealed.

*In the x 10 group.* No lesions were revealed.

*Clinical and biochemical*

Blood test: hemoglobin level; erythrocytes, leucocytes, and thrombocytes counts: within the physiologic norm in all groups. The experimental groups results showed no significant differences from the control intact groups and comparison drug.

*Biochemistry:* blood glucose, alanine and asparagine aminotransferases activity normal in all groups.

**Conclusion.** The original pharmaceutical composition was subchronic toxicity tested in experimental immature animals.

It has been revealed that the substance intranasal administration, in both the therapeutic and ten-fold therapeutic doses, in the course of 2 weeks, provokes no viscera alteration and gives no toxic effect on the blood system. No local irritant effect has been revealed.

**Example 6. Pharmaceutical composition mutagenicity.**

To evaluate the claimed protein-polypeptide pharmaceutical composition mutagenicity, the following set of tests was used:

- Ames Salmonella/microsome gene mutagenicity assay using the TA 97, TA 98, and TA 100 *Salmonella typhimurium* strains as test objects; and
- micronuclei in murine bone marrow cells.

A protein-polypeptide pharmaceutical composition sample was tested, in form of transparent liquid sterile packed in a dark glass vial, 20 ml, 0.1 mg/ml substance.

**Ames Test Protein-Polypeptide Pharmaceutical Composition Mutagenicity Assessment.**

The *Salmonella typhimurium* mutagenicity assay is a bacterial test system to account for the histidine prototrophy gene mutations under effect of the chemical compounds and/or their metabolites, inducing the mutations of the base substitution or reading frame shift in organism genome type. The mutagens inducing the base pairs substitution are the agents provoking the base pairs substitution mutations in the DNA molecule. The mutagens inducing the genetic code reading frame shift mutations are the agents provoking the addition or deficit of single or multiple base pairs in the DNA molecule.

The method's purpose is to reveal the capability of the pharmaceutical substances or their metabolites to induce the gene mutations in the *Salmonella typhimurium* indicator strains. The bacteria are treated with a compound under test with (SM+) or without (SM-) metabolic activation system. After a certain time incubation, the different test strains revertant colonies number is compared with that of the negative control spontaneous revertant (untreated or solvent treated cultures). If the compound under test and/or its metabolites have mutagenic activity, they will induce the reverse mutations from histidine auxotrophy to prototrophy in the *Salmonella typhimurium* histidine-dependent strains.

The tests used a set of the *Salmonella typhimurium* indicator strains allowing to register the genetic code reading frame shift (TA 98 and TA 97) base pair substitution (TA 100) mutations. The strains originate from the Russian National Collection of Industrial Microorganisms, Research Institute for Genetics and Selection of Industrial Microorganisms, Scientific Center of Russian Federation. The bacterial cultures handling Rules of procedure, the strains genotype checks, their museum keeping rules, the experimental utensils, reagents, nutrient media and solutions, rat liver homogenate getting, activating mix compounding, and statistical analysis methods complied with the standards described in detail in the relevant literature.

The metabolic activation used the S9 fraction of the Wistar male rats liver; 5 days before sacrifice the rats received the microsomal enzymes inductor (sovol, 300 mg/kg, single, intraperitoneally). To control the metabolic activation system the ethidium bromide (10 µg/dish), TA 98 strain, SM+, was used.

The 2.3 mg/ml protein sterile protein-polypeptide pharmaceutical composition sterile solution was examined: 1.0 and 0.3 ml/dish mother solution and three 10X dilution in physiological solution (0.1 ml/dish). The substance test doses were 300, 100, 10, 1, and 0.1 µg/dish.

The experiment had positive controls; for them the mutation-inducing substances in relevant microorganisms strains with or without activation were used. The no-activation variants used the sodium azide, 10 µg/dish, TA 100 strain, SM-; 2,7-2,7-diamino-4,9-dioxy-5,10-dioxo-4.5,9,10-tetrahydro-4,9-diazopyrene (DDDTDP), 10 µg/dish, TA 98 strain, SM-; and 9-aminocaridine (9AA), 50 µg/dish, TA 97 strain, SM-. To control the metabolic activation system activity the ethidium bromide (10 µg/dish), TA 98 strain, SM+, was used. The negative control used the physiological solution (0.1 ml/dish).

The selective semi-enriched agar (0.7%) in test tubes was melted in water bath at 100°C; then it was placed into the 45-46°C thermostated water bath.

First, 0.1 ml of the relevantly diluted sample, then 0.1 ml of the bacterial suspension and 0.5 ml of the microsomal activating mix (metabolic activation variant) were put into the agar test tubes. Then the tube content was quickly blended and discharged onto the lower minimal agar in the Petri dishes. The microsomal activating mix and semi-liquid agar reserve introduction time per dish did not exceed 10 to 15 seconds. The dishes were left for 30 to 40 minutes at ambient temperature; after the first agar gelation they were put into a 37°C thermostat. The results were accounted after a 48-hour incubation.

The no metabolic activation system (SM-) and metabolic activation system (SM+) variants were experimented in parallel. In the SM- variant the direct mutagens (drugs mutagenic due to the compound initial structure activity) effect may be observed. As of the effect of the promutagens, i.e., the compounds whose effect associates with the mutagenic metabolites formation, this may be accounted when comparing the results of the SM- and SM+ compounds tests analysis data. Every control and experimental variants used 2 dishes each. The mutagenic effect was considered significative when the mean number of the revertants colonies per dish in experiment exceeded the control one 2- and more fold. The experimental results were taken into account at the condition of standard response in all the positive and negative control variants. The revertants colonies numbers in the solvent control, SM- and SM+ variants, were within the spontaneous level fluctuations range for the given strains. The strains response to the standard mutagens was within the common levels range.

The examined with all concentrations protein-polypeptide complex has not shown any mutagenic effect for the TA 100, TA 98, and TA 97 strains, with and without metabolic activation system.

CONCLUSION: the protein-polypeptide pharmaceutical composition is unable to induce gene mutations with the *Salmonella typhimurium* test strains within all the experimental concentrations range.

**Micronuclear Test Protein-Polypeptide Composition Mutagenicity Assessment in Mammalia Cells.**

The cytogenetic activity is the capacity of a substance to provoke structural and quantitative chromosomal abnormalities in the somatic and embryonic cells.

The micronuclei are the small size DNA-containing formations consisting of the chromosomal acentric fragments or lagged in ana-telophase chromosomes. At the telophase stage these fragments may get into the daughter cells nuclei or form individual or multiple micronuclei in cytoplasm.

The Study purpose is to reveal and quantitatively assess the cytogenetic activity (mutagenicity) of the pharmacologic drugs in the mammalian bone marrow polychromatophils. The method's base is the micronuclei cells microscopic registration.

The experimental used the male and female F1(CBAx C57BI6/J) hybrid mice, two-month age, 18 to 20 grams body weight. Every group included 6 animals. The animals were kept under the 12-hour light regime, with freely available water and food. The single and five fold administration protein-polypeptide complex mutagenicity assessment animal experiments were in compliance with the official protocols. Three experimental series with relevant controls were performed: therapeutic dosage: males, single administration; subtoxic dose: males, single administration; and therapeutic dosage: males and females, five fold administration.

Taking into account that the possible biologically active protein-polypeptide pharmaceutical composition clinical administration method may be intravenous or endolumbar, this Study used the intraperitoneal one with mice. The protein-polypeptide complex mutagenicity assessment dose was defined on the basis of the maximum recommended daily therapeutic dose (TD) for human. The 2 ml at 0.1 mg/ml concentration IV administration is recommended. The therapeutic dose for human, including infant, may be 0.05 mg/kg/day. To calculate the mice experimental dose, the conversion factor is recommended to take into account the human and murine body surface area to body mass ratio. In our study it was 8.33. Hence, for the murine therapeutic dose the approximately 8.33 human therapeutic dose was taken (0.42 mg/kg).

For the subtoxic dose the maximum possible experimental one was used, 10 mg/kg, as the substance concentration was 0.1 mg/ml; the conventional total substance administered to mice is 0.1 ml per 10 g body weight. Hence, calculated for human, the maximum tested dose was 1.7 mg/kg.

Seven groups of animals, four experimental and three control ones, were formed. The 0.1 mg/kg substance was five times, at 24-hour intervals, administered to both the males and females. In all the groups the animals were sacrificed 6 hours after the last protein-polypeptide complex administration.

In two other experimental groups, the protein-polypeptide complex was administered once to the males, at 0.2 mg/kg and 0.5 mg/kg.

For the negative control the negative control the physiological solution was used. It was administered intraperitoneally to the males and females of the two control groups, the times and volumes were the same as for the experimental. Positive control: 20 mg/kg cyclophosphamide dissolved ex tempore in physiological solution, single intraperitoneal administration, 24 hours before sacrifice.

The bone marrow cells preparation for the micronuclei account were made in conventional manner, in accordance with the "Micronuclear Test Environmental Factors Mutagenicity Assessment in Mammalia Organs Cells" Guidelines. The sacrifice method was the cervical dislocation 6 hours after last drug administration. Both femoral bones were extracted, muscles were then removed. To wash out the bone marrow the bovine embryonic serum (NPP PanECO). The bone marrow from the both femoral bones was extracted into a microtube with a syringe. The suspension was centrifuged at 1000 rpm, 5 minutes; the supernatant was removed, the sediment was carefully resuspended. A drop of the suspension was used to prepare the smear then dried in air. The smear was then methanol fixed during 3 minutes. For staining the Romanowsky-Giemsa method was used. 2000 polychromatophils per animal were then analyzed with coded preparations. At the 500 erythrocytes count the polychromatophil to normochromatic erythrocytes ratio was determined. Analysis finished, the preparations were coded.

The positive result criterion is the reproducible and statistically significant growth of the micronuclei polychromatophil erythrocytes number in at least one experimental group compared with the control one. The positive result indicates that the substance induces the chromosomal damages and/or cells mitotic apparatus damages in experimental animals.

The micronuclei account results statistical treatment used the X2 criterion experimental to control series comparison; the polychromatophils proportion intergroup comparison used the Mann-Whitney criterion.

Mice bone marrow micronuclei polychromatophils account results: in no experimental variants the protein-polypeptide pharmaceutical composition induced the mice bone marrow micronuclei polychromatophils proportion augmentation as compared between the experimental and relevant control series. The 20 mg/kg cyclophosphamide (positive control) intraperitoneally in male mice provoked 19.6 fold higher micronuclei cells (62.1‰ vs. 3.16‰ in control,

P < 0.001). The polychromatophil erythrocytes share in the total bone marrow erythrocytes was at approximately the same level for both the experimental and control groups; hence, no toxic effect of the substance in tested doses on hematogenesis. Under the positive control (cyclophosphamide) the polychromatophil to normochromatic erythrocytes ratio shift to the latter ones was recorded (compared with control, P < 0.005).

Thus, the test for the micronuclei induction in the mice bone marrow micronuclei polychromatophils at one and five fold 0.42 mg/kg body weight intraperitoneal administration to males and females (corresponding, as recounted, to the daily therapeutic dose for human) has not revealed any protein-polypeptide complex mutagenic activity. No mutagenic activity has been revealed for the maximum possible, 10 mg/kg, single protein-polypeptide pharmaceutical composition administration in males. The protein-polypeptide complex has not shown any toxic effect as of the "polychromatophil erythrocytes proportion" parameter.

CONCLUSION FOR THE PROTEIN-POLYPEPTIDE PHARMACEUTICAL COMPOSITION MUTAGENICITY ASSESSMENT

The protein-polypeptide pharmaceutical composition has shown no mutagenic activity in either the Ames Salmonella/microsomes test or the mice bone marrow cells micronuclei induction test performed in accordance with the Guidelines for Experimental (Preclinical) Study of New Pharmacological Substances.

**Example 7. Effect of pharmaceutical composition rats survival rate under brain ischemic damage at both carotid arteries ligation (incomplete global ischemia of brain).**

The experimental used the Wistar male rats, 180 to 250 g body weight, under irreversible bilateral occlusion of the common carotid arteries [3-5]. The laboratory animals received from nursery were 5 days kept in quarantine. All the veterinary manipulations were aseptic, to keep the experimental animals status. The experimental animals were kept in standard conditions [6]. The rats received the ether (chemically pure diethyl ether, OAO "Medhimprom") anesthesia; then a 8 to 10 cm long skin discission was made in the neck region, along the median line, to access the carotid arteries. The both carotid arteries were exposed, separating the vagus and sympathetic nerves. The silk threads (Silk blk 17 x 45 cm/M3/USP2/ Non needled/ Ethicon Johnson & Johnson, cat. No W203) were pulled under the vessels; then, together with an assistant, the both carotid arteries were single-step ligated with triple friction knot. The skin discission was then sutured with the surgical silk (Silk blk 100 cm/M5/Sgle armed KP-3USP2/ Non needled/ Ethicon Johnson & Johnson, cat. No W794), one or two surgical stitches, with further wound treatment with the 5% iodine tincture. The physiological solution or pharmaceutical composition were administered intraperitoneally. The total manipulations time was 8 to 10 minutes; then the animals were placed into cage. Three groups of animals were used:

- **Group 1:** control animals, physiological solution with sterile disposable insulin syringe: 0.5 ml/rat in 1 hour and 0.5 ml/rat in 5 hours after carotid arteries ligation;
- **Group 2:** sham operated animals: same manipulations as in control but without carotid arteries ligation; physiological solution 0.5 ml/rat in 1 hour and 0.5 ml/rat in 5 hours; and
- **Group 3:** animals after carotid arteries ligation; biologically active substance: 0.5 ml/rat 0.1% solution (0.2 mg/kg) in 1 hour and 0.5 ml/rat in 5 hours.

The survival rate was followed up immediately after the procedure and in the course of the first day, i.e., the final survival rate was fixed in 24 hours. The drug biological activity in form of the brain protection under incomplete global ischemia at single-step carotid arteries ligation was assessed as follows:

- **expressed:** survival rate: > 80% rats;
- **median expressed:** survival rate: 70% to 80% rats; and
- **unexpressed:** survival rate: < 70% rats.

The data statistical processing used the Fischer exact test, accounting for the comparisons multiplicity.

The experiments have shown that the greatest number, i.e., 11 of 24, of the animals in control died within the 1st hour after the carotid arteries ligation; 4 more animals died within the next 24 hours; in total, 15 rats of 24 died (63%, see Table 1). Among the sham operated animals 2 (17%) rats died immediately after the operation. The pharmaceutical composition 0.2 mg/kg (0.5 ml 0.1% solution) intraperitoneally in 1 and 5 hours provoked death in only two animals: 34/36 rats survived. The compared to control survival rate augmentation is 95%. Table 1 shows the obtained data.

**Table 1. Effect of pharmaceutical composition on rats survival rate under ligation of both carotid arteries**

| Groups of animals | Expressed bioactivity | |
| --- | --- | --- |
| | Survived | Died |
| Group 1<br>Control: 24 rats | 9 of 24 (37%) | 15 of 24<br>63% |
| Group 2<br>Sham operated<br>12 rats | 10 of 12* (83%) | 2 of 12 (17)% |
| Group 3<br>Pharmaceutical composition 0.2 mg/kg<br>36 rats | 34 of 36* (95%) | 2 of 36 (5%) |
| * -P $\leq$ 0.01 compared with control. | | |

Hence, the pharmaceutical composition ensures a statistically significant animals survival rate augmentation compared with control under incomplete global ischemia of brain at single-step both carotid arteries ligation.

The Study has revealed that the claimed pharmaceutical composition has the expressed neuroprotective and neurometabolic effects stimulating the nervous tissue physiologic and reparative regeneration; its biological activity is higher than that of the Cerebrolysin reference drug.

The claimed pharmaceutical composition may find application in the treatment of the vascular, traumatic, toxic, hypoxic, and autoimmune genesis central and peripheral nervous system diseases.

**Example 8. Study of pharmacologic composition effect on local cerebral blood flow in anesthetized rats brain cortex under global transient ischemia.**

The experimental used the anesthetized Wistar line male rats, 220 to 250 grams body mass, natural respiration. The anesthesia used the urethane (Sigma Aldrich Chemie GmbH, Germany), 1400 mg/kg, intraperitoneally.

**Technique.** The brain blood circulation in animals was assessed using the rats brain cortex local blood flow Doppler laser flowmetry records. The local blood flow in the rats brain cortex parietal region was recorded with the ALF-21 flowmeter (Transonic System Inc., USA). For this, the 0.8 mm diameter flowmeter needle sensor was installed in the rat brain cortex parietal region with the help of a micromanipulator and beam. At the same time, the arterial blood pressure fluctuations were recorded using a polyethylene catheter preinserted into the femoral artery. The blood flow and arterial blood pressure readings were recorded by the BIOPAK (USA) polygraph and personal computer. The 0.2 mg/kg (0.5 ml 0.1% solution) pharmacologic composition was administered into the femoral vein via the polyethylene catheter.

The global transient ischemia model is widely used to study both the cerebral blood flow disturbances and the different post-ischemic brain function biochemical alterations. The global transient ischemia in rats was provoked by the 10-minute occlusion of the both common carotid arteries with the help of special clamps; at the same time, the arterial pressure was reduced down to 40 to 50 mm Hg by pumping out the arterial blood via the polyethylene catheter preinserted into femoral artery. In 10 minutes, the clamps were removed from the carotid arteries; the earlier taken blood was returned to the animal [3-5]. The test results were variance analysis treated using for the remeasurements the disperse analysis and the Dannett's multiple comparison test and Biostat software.

**Results of Study**

The experiments have shown that the rats brain cortex global transient ischemia provokes the local brain blood flow downswing, which downswing is, in average, $6.1\pm1.0$ RVU or $82\pm2.2\%$ of the initial level. Having removed the clamps from the carotid arteries, and reinfused the blood, a blood flow upswing is observes, which gradually reduces, and, in 30 minutes after reperfusion, in average, $25\pm2.8$ RVU or $23\pm2.4\%$ of the initial level ($p < 0.05$).

The pharmacologic composition administered in 30 minutes after the 0.2 mg/kg (0.5 ml 0.1% solution) reperfusion induced, in most cases, already 10 minutes after administration, the rats brain cortex local blood flow augmentation by, in average, $12 \pm3.3\%$ (7/10 experiments). The start of the statistically significant augmentation of the local cerebral blood flow was on the 50th minute, the average augmentation was $39\pm9.2\%$, and such augmentation continued until the 90th minute of observation (se Table 2). It should be noted here that in the physiological solution controls the local cerebral blood flow level remains practically the same.

The arterial blood pressure was measured in parallel with the cerebral blood flow. The experimental has shown that 10 minutes after the pharmacologic substance administration to rats the gradual arterial blood pressure decrease was observed; the arterial blood pressure decreased in 50 minutes by, in average, $15\pm3.4\%$; and this hypotension remained up to the experiment completion (see Table).

It should be noted that the physiological solution controls have also shown a small arterial pressure decrease, by, in average, 18%; hence, the pharmacologic substance has no significant effect on the arterial pressure level.

**Conclusion**

Hence, the Study has shown that the pharmacologic substance improves the experimental animals survival rate under carotid arteries complete ligation. At the same time, the pharmacologic substance increases the rats brain cortex local blood flow, reduced after the brain global transient ischemia; to the end of the experimental, this flow is greater than the pre-ischemia control. It may be that the ligated carotid arteries animals survival rate augmentation under effect of the pharmacologic substance may be due to its capacity to improve the experimental animals brain cortex blood supply.

**Example 9. Pharmaceutical composition neuroprotective effects at in vitro glutamate toxicity.**

The *in vitro* test of the biologically active protein-polypeptide complex pharmaceutical composition neuroprotective capacities at glutamate toxicity used the 7-day cell cultures: Wistar line rats cerebellar granule cells obtained in enzymatic mechanic dissociation. Brain tissue dissociation method: the extracted cerebella were placed in a plastic Petri dish filled with phosphate buffer; then these were washed with the same solution several times and fragmented. The tissue fragments were 15-minute incubated at 37°C in the phosphate buffer based enzymatic mix with 0.05% trypsin and 0.02% EDTA. After incubation the tissue was washed twice in phosphate buffers and once in cultivation medium; then it was mechanically dissociated in the cultivation medium. The nutrient medium contained: 10% bovine embryonic serum, 2 mM glutamine, and 10 mM HEPES buffer, pH 7.2-7.4. The cells suspension was 1-minute centrifuged at 1000 rpm; the supernatant was discharged; the sediment was resuspended in the nutrient medium where the K+ concentration had been brought to 25 mM, which has a trophic effect on the cerebellar granule cells. The cultivation went on in the 96-socket plastic cameras. Into every socket, 0.1 ml cells suspension was added. The cultures developed in a $CO_2$ incubator, at 35.5°C, 98% RH.

To the 7th cultivation day, upon the glutamate receptors maturity, the 15-minute glutamate toxic treatment (25, 37, 50 $\mu$M, 15 minutes) was performed, in the balanced saline solution (mM: 154 NaCl, 25 KCl, 2,3 $CaCl_2$, 1 $MgCl_2$ 3,6 $NaHCO_3$, 0,35 $Na_2HPO_4$ 10 HEPES (pH 7.6)); then the preparation was washed twice; the third solution contained the pharmaceutical composition (0.05 and 0.1 mg/ml). Control (same volume): $H_2O$ glycin buffer, and bovine serum albumin (0.15 mg/ml). All was then 4-hour $CO_2$-incubated to develop the neurodegeneration. Then the mix was 20-minute fixed with the formalin - alcohol - acetic acid 2:7:1 mix, stained with the trypan blue, and filled with glycerol; the photos were shot by a digital camera.

The neurons viability was assessed by counting the normal morphology neurons on the fixed trypan blue stained preparations encapsulated in glycerol. Every point used 3 sister cultures; the cells were counted in 5 culture visual fields, x 40 lens. The survival rate was calculated as percentage of control.

The protective effect is more illustrative having calculated the protection efficiency factor (PEF) from the formula:

$$PEF = (N_O - N_B)/N_O \bullet 100\%,$$

where: $N_O$: mean percentage of the neurons loss due to the noci-influence (here, glutamate) in the $H_2O$ addition cultures; and $N_B$: mean percentage of the neurons loss in the neuron pharmaceutical composition addition cultures. This parameter allows for comparing the efficiencies of different substances with different ischemia models.

## Results of Study

**Series 1.** The cerebellum dissociated cultures consist of the neurons and neuroglials. The neuronal population is represented by the granule cells as the other larger size cerebellum neurons are already differentiated to the dissociation moment, and, hence, do not survive the procedure. The granule cells form the largest class of the brain neurons; they are morphologically and neurochemically homogeneous. To day 7 of cultivation the glutamate receptors become mature. The 3 to 5-hour exposure to the balanced saline solution with the glycin buffer (GB), bovine serum albumin (BSA), and 0.05 and 0.1 mg/ml pharmaceutical composition added has not led to change of the living neurons number compared with control.

The 25, 37, and 50 $\mu$M (15 minutes) glutamate provoked the dose-dependent death of the murine cultivated cerebellar granule cells.

To reveal the protective effects of different substances the glutamate induced death of cells must not exceed 70%. Thus, the neuroprotective experimental used the 25 $\mu$M glutamate concentration. Both the 0.1 and 0.05 mg/ml pharmaceutical compositions improve the protective effect, increasing the cerebellar granule cells survival rate from 54.97+6.5% to 72.76+9.7%, respectively. However, this effect is more expressed at a lower concentration.

In our event, for the 0.05 mg/ml pharmaceutical composition, REF = (45.03-27.24)/45.03 • 100% = 39.51%; 0.1 mg/ml: REF =(45.03-37.85)/45.03 • 100%=15.94%

The glycin buffer and bovine serum albumin added in the post-glutamate period did not change the glutamate toxicity. In whole, the first experimental series counted about 2,000 cells from 60 sister cultures.

**Series 2.** The second series used the same experimental techniques and patterns as the first one, except that the pharmaceutical composition used two, 0.05 и 0.01 mg/ml, concentrations, and the Cerebrolysin in the same concentrations was used for reference drug. The control calculation data show that the 3 to 4-hour exposure to the balanced saline solution with the 0.05 and 0.01 mg/ml glycin buffer, pharmaceutical composition, and Cerebrolysin additions have not led to change of the living neurons number compared with control.

The 25, 37, and 50 $\mu$M (15 minutes) glutamate provoked the dose-dependent death of the murine cultivated cerebellar granule cells. To reveal the protective effects of different substances the glutamate induced death of cells must not exceed 70%. Thus, the neuroprotective experimental used the 25 $\mu$M glutamate concentration. The observed similar dose dependent surviving neurons score in function of the glutamate concentration at 35 and 50 $\mu$M indicated the receptors saturation. The pharmaceutical composition had a reliable protective effect, improving the cerebellar granule cells survival rate This effect if dose dependent: maximum protection at maximum biologically active protein-polypeptide complex concentration in pharmaceutical composition (0.05 mg/ml). Here, REF =(45-20)/45 • 100%=55.6%.

For 0.01 mg/ml: REF =(45-36)/45 • 100%=20%

The tested dosages Cerebrolysin had no reliable protective effect (REF = (45-38)/45 • 100%=15.6%, at 0.05 mg/ml; and REF = (45-42)/45 • 100%=6.7%, for 0.01 mg/ml).

The post-glutamate period added glycin buffer even increased somehow (non-reliable) the glutamate toxicity.

**Pharmaceutical composition aggregate protective affect as of the experimental series:**

The above results of individual experiments show that the pharmaceutical composition protective effect after the toxic exposure to glutamate is stable, experiment-to-experiment reproducible. The drug itself has shown no toxic effect in any experiment. Neurons death rate and protection factors: 3 hours after the 25 $\mu$M glutamate exposure 41.49+4% neurons are lost; if after the glutamate exposure the presence in the saline solution of the 0.5 or 0.1 mg/ml pharmaceutical composition reduced the cultivated cerebellar neurons death rate down to the respective 30.88+4.3 and 21.98+4.37. With this, this glutamate toxicity reduction at the 0.5 or 0.1 mg/ml pharmaceutical composition introduction after lesion was reliable (n = 45, P < 0.05, Anova tow-way test with Benferroni post-test). Moreover, for the 0.1 mg/ml pharmaceutical composition, the trend was well expressed.

Protection efficiency factors (PEFs) for various concentrations:

$$REF\ (0.1\ mg/ml) = (41.49\text{-}30.88)/41.49 \bullet 100\% = 25.57\%;$$

$$REF\ (0.05\ mg/ml) = (41.49\text{-}21.23)/41.49 \bullet 100\% = 48.83\%;$$

and

$$REF\ (0.01\ mg/ml) = (41.49\text{-}21.98)/41.49 \bullet 100\% = 47.02\%.$$

It should be noted that the above 80% or below 30% glutamate toxicity experimental results were neglected.

The tested dosages Cerebrolysin had no reliable protective effect (REF = (45-38)/45 • 100%=15.6%, at 0.05 mg/ml; and REF = (45-42)/45 • 100%=6.7%, for 0.01 mg/ml).

The post-glutamate period added glycin buffer even increased somehow (non-reliable) the glutamate toxicity.

**Example 10. Study of pharmaceutical composition pharmacologic effects mechanisms.**

The pharmaceutical composition specific effect on the excitatory and inhibitory neurotransmitters concentrations dynamics was studied *in vitro,* in the 7-day cerebellar granule cells cultures intercellular fluid, under glutamate cytotoxic effect. The drugs affecting the glutamate release (Lubelizil, BW619C89), are known to be efficient neuroprotectors.

**Technique.** The assay was taken of 50 $\mu$l of the cerebellar granule cells cultivation medium, before and after the glutamate toxic exposure, and pharmaceutical composition, glycin-phosphate buffer, and/or physiological solution addition. The resulting media samples were frozen at 20°C, coded, and sent to laboratory to measure the neurotransmitters concentrations in the high efficiency liquid chromatography with electrochemical detection. The LC-4B (BAS, USA) at +850 mV potential on glass carbon electrode vs. reference Ag/AgCl electrode was used. The sodium phosphate 0.05 M buffer with 0.025 mM EDTA and 5% acetonitrile served as mobile phase. To derive the amino acids, to 25 $\mu$l perfusate, 25 $\mu$l 0.01 mg/ml L-homoserine internal standard in 0.2 n NaOH and 10 $\mu$l #-phtalal-dehydsulfite agent in 0.1 M borate buffer (pH 9.5). For standard, the solution was used containing the amino acids mixture in 0.01 mmol/l concentration in 0.1 n HClO$_4$ glutamate in 0.2 mg/ml concentration in 0.1 n HClO$_4$. After the 15-minute 37°C incubation, the mixes were applied on the Agilent Hypersil ODS 5 $\mu$m, 4.6 x 250 (5 $\mu$l loop volume) of the Agilent 1100 (USA) chromatograph. The amino acids concentration was calculated with the Chemstation Agilent (USA) software; the end result was expressed in nM/mg of tissue in 2 minutes.

The resulst were statistically analyzed by the Biostat software, using the non-parametric criteria (Wilcoxon-Mann-Whitney U-test).

The mobile phase consisting of 0.069 M anhydrous KH$_2$PO$_4$ (Fluka), 0.27 M citric acid monohydrate (Fluka), 0.27 mM ethylenediaminotetraacetic acid sodium salt (Sigma), and 1.9 mM sodium octylsulfonate (ion-pair reagent) (Diapharm) was prepared as follows: the batch weight was diluted in 920 ml deionized water, pH was brought to 3.15 (pH-340 pH-meter (ZIP)), then 80 ml, i.e., 1.528 M acetonitrile (Merck) was added. The solution was vacuum filtered (0.2 $\mu$m microfilter) under 20 to 40 mm Hg pressure. Before the biochemical experiments the mobile phase was 40 to 50-second degasified under vacuum with simultaneous ultrasonic bath treatment (Serga, Russia).

Results: the 0.05 and 0.1 mg pharmaceutical composition reliable and dose-dependently augmented the glutamate, glycin, gamma-aminobutyric acid, taurin, and aspartate extracellular release (see Table).

| Experimental series | Tested neurotransmitters ($\mu$Mol/l) | | | | |
|---|---|---|---|---|---|
| | Asp | Glu | Gly | GABA | Tau |
| Control | **0.0008 +** 0.00015 | **0.00014 +** 0.00067 | **0.011 +** 0.0048 | **0.00023 +** 0.00013 | **0.038 +** 0.0091 |
| 0.05 mg pharmaceutical composition | **0.0011 +** 0.00052 | **0.0003 +** 0.000083 | **0.56** + 0.224 | **0.00047 +** 0.0003 | **0.0635 +** 0.029 |
| 0.1 mg pharmaceutical composition | **0.0011 +** 0.0005 | **0.00034 +** 0.00012 | **0.702** + 0.39 | **0.0004 +** 0.00022 | **0.075** + 0.034 |
| Control with 25 $\mu$M glutamate toxicity | **0.0009 +** 0.0004 | **0.00021 +** 0.00013 | **0.0516 +** 0.0417 | **0.00021 +** 0.00012 | **0.0457 +** 0.02023 |
| 0.05 mg pharmaceutical composition with 25 $\mu$M glutamate toxicity | **0.00075 +** 0.000067 | **0.00027 +** 0.00006 | **0.43341 +** 0.22624 | **0.00037 +** 0.00016 | **0.0651 +** 0.01613 |
| 0.1 mg pharmaceutical composition with 25 $\mu$M glutamate toxicity | 0.0007 + 0.00002 | **0.00026 +** 0.000153 | **0.7398 +** 0.24536 | **0.00056 +** 0.00033 | **0.07977 +** 0.017958 |

At the 0.05 mg pharmaceutical composition concentration the neurotransmitters release level was by 20% higher ($p < 0.05$), at 0.1 mg the also certain augmentation was 28%. After the 25 $\mu$M glutamate toxicity, and 0.05 mg and 0.1 mg pharmaceutical composition, the dose-dependent GABA, as inhibitory neurotransmitter, augmentation was observed; it made 90% and 160%, respectively, in relation to the control ($p < 0.01$).The taurin and glycin concentrations dose dependently grew to the same level, both with and without glutamate toxicity. The excitatory neurotransmitters, aspartate and glutamate, at glutamate toxicity and pharmaceutical composition reduced to the control for aspartate ($p < 0.05$) and remained unchanged with glutamate, without certain distinction from its effect without glutamate toxicity.

Hence, the pharmaceutical composition physiologic effect showed itself in the higher glycin, glutamate, taurin, and GABA concentrations. This effect intensity depended on the pharmaceutical composition concentration, reliably higher at 0.1 mg than at 0.05 mg.

Taken into account sources of information:

1. Encyclopedia of Drugs, 2006, p. 970.

2. RF Patent No 2128511(1999)

3. RF Patent No 2104702(1999)

4. RF Patent No 2049472(1999)

5. Guidelines for Experimental (Preclinical) Study of New Pharmacological Substances. General editor: Prof. R.U. Khabriev, RAMS. 2nd ed., rev. and corr. Moscow, OAO "Meditsina" Publishing House. 2005., p.131-170.

6. Methodological Guidelines. Carcinogenicity Assessment of Pharmacological Drugs. // Guidelines for Experimental (Preclinical) Study of New Pharmacological Substances. General editor: Prof. R.U. Khabriev, RAMS. 2nd ed., rev. and corr. Moscow, OAO "Meditsina" Publishing House. 2005., p.100-122.

7. Methodological Guidelines. Carcinogenicity Assessment of Pharmacological Drugs and Excipients in Short-Term Tests. // Guidelines for Experimental (Preclinical) Study of New Pharmacological Substances. General editor: Prof. R.U. Khabriev, RAMS. 2nd ed., rev. and corr. Moscow, OAO "Meditsina" Publishing House. 2005., p.131-170.

8. I.V. Silkina, T.S. Gan'shina, S.B. Seredinin, P.S. Mirzoyan. Enhancement of Blood Supply to Ischemic Brain Under Effect of Afobazol. J. Exp. and Clin. Pharmacology, 2004, vol. 67, No 5, p.9-13.

9. Smith M.L., Bendek G., Dahlgen N. at all, Models for studing, long-term recovery following forebrain ischemia in the rat. A 2-vessell occlusion model.// Neurol.Scan., 1984, v.69, p.385 - 400.

10. Φ Y.Wang-Fisher. Manual of Stroke Models in rats. CRC press, 2008, p.3-4

11. Laboratory Animals Care and Keeping Program. Nursery for laboratory animals under Federal Institute of bioorganic chemistry, November 2005.

## Industrial Applicability

The pharmaceutical composition effect on the glutamate toxicity model background compared with the physiologic effect without glutamate toxicity showed itself in:

- reduction of aspartate extracellular level;
- significant, dose dependent augmentation of GABA level;
- stabilization of glycin and taurin concentrations; and
- trend to glutamate level decrease.

The pharmaceutical composition pharmacologic effect (higher granule cells survival rate in cerebellar cells culture under glutamate cytotoxic effect) is due to the drug regulatory effect on the excitatory and inhibitory mediations levels, ensuring the necessary protection of the neurons against damage. The most interesting effect of the pharmaceutical composition action on the amino acids extracellular pool change is, in addition to the GABA concentration growth, the taurin level augmentation. This amino acid, being SH-groups donator, increases the glutathione-peroxidase system effect, improving thus the neurons cellular membranes antioxidant protection. The used glutamate toxicity model allows to assess the pharmaceutical composition effect on the dynamics of both the neurotransmitters extracellular level, including the amino acids metabolic pool, and the synaptic pool (indirectly). In other words, the pharmaceutical composition neuroprotective effect is explained by the glutamate level stabilization, with the trend to reduction due to both the neurotransmission inhibitory component activation and the excitatory neurotransmitters spillover decrease.

## Claims

1. Pharmaceutical composition for use in the treatment of vascular, traumatic, toxic, hypoxic, and autoimmune genesis central and peripheral nervous system diseases, in the form of the solution for parenteral, intranasal, and subconjunctival administration; wherein the active ingredient is the 0.01 to 2.0 mg/ml biologically active protein-polypeptide complex with the tissue-specific reparative effect on the nervous tissue, obtainable from the mid first third to mid last third gestational age livestock quickly frozen embryonic brain tissue, which complex contains the negatively charged faintly acid neutral proteins and polypeptides relating to the growth factor, differentiation factor, signalling molecules, that determine its biologic and pharmacologic activity, with the 5 to 200 kDa molecular masses, wherein at least 80% of the total protein mass has the 10 to 120 kDa molecular mass, and **characterized by** a peak at the 274-284 nm wave length in the UV-visible spectrum and bands in the pI 4.2 to 8.4 range at isoelectric focusing in a 5% polyacrylamide gel, and which complex contains a pharmaceutically acceptable diluent.

2. Pharmaceutical composition for use according to Claim 1, wherein the diluent is an officinal buffer solution with excipients, including the high molecular weight compounds, stabilizers, preservatives, and solubilizers.

## Patentansprüche

1. Pharmazeutische Zusammensetzung zur Verwendung in der Behandlung von vaskulären, traumatischen, toxischen, hypoxischen und autoimmungenen Erkrankungen des zentralen und peripheren Nervensystem, in Lösungsform zur parenteralen, nasalen- und subkonjunktivalen Verabreichung, wobei der Wirkstoff ein biologisch aktiver Protein-Polypeptidkomplex mit einer Konzentration von 0.01 bis 2.0 mg/ml ist, der einem gewebespezifischen reparativen Effekt auf das Nervengewebe hat und erhältlich ist aus schockgefrorenem Hirngewebe eines behuften Nutzviehembryos eines Gestationsalters von der Mitte des Ersttrimesters bis zur Mitte des Letzttrimesters, wobei der Komplex negativ geladene, leicht saure, neutralen Proteine und Polypeptide umfasst, die Wachstums-, Differenzierungsfaktoren und Signalmolekülen zuzuordnen sind und seine Bio- und Pharmaaktivität bestimmen, mit einer Molekülmasse von 5 bis 200 kDa, wobei mindestens 80% der Gesamtproteinmasse eine molare Masse von 10 bis 120 kDa hat, und durch einen Maximalwert der Wellenlänge von 274-284 nm im UVsichtbarem Spektrum und durch die Präsenz von Banden im pI Bereich von 4.2 bis 8.4 bei isoelektrischer Fokussierung im 5% Polyacrylamidgel gekennzeichnet ist, und wobei der Komplex ein pharmazeutisch akzeptables Lösungsmittel umfasst.

**2.** Pharmazeutische Zusammensetzung zur Verwendung gemäß Anspruch 1, wobei das Lösungsmittel eine offizielle Pufferlösung samt Exzipienten, umfassend die Verbindungen mit hoher molarer Masse, Stabilisatoren, Konservierungsmitteln und Lösungsvermittlern, ist.

**Revendications**

**1.** Composition pharmaceutique destinée à l'utilisation pour le traitement des maladies vasculaires, traumatiques, toxiques, hypoxiques et auto-immunes du système nerveux central et périphérique en forme de solution à administration parentérale, intranasale et sous-conjonctivale; dont la substance active est un groupe complexe protéino-polypeptidique biologiquement actif d'une concentration de 0,01 à 2,0 mg/ml, exerçant un effet spécifique régénérateur sur le tissu nerveux, obtenue à partir du tissu cérébral surgelé d'embryons d'animaux agricoles congelés d'âge in utero entre le milieu du premier trimestre et le milieu du dernier trimestre, contenant des protéines et polypeptides négatogènes neutres d'une faible acidité par rapport au facteur de croissance et de différenciation aux molécules de signal définissant son activité biologique et pharmaceutique, dont la masse moléculaire est de 5 à 200 kDa, alors qu'au moins 80% de la masse globale protéique a une masse moléculaire entre 10 et 120 kDa, **caractérisé par** une valeur maximale de longueur d'onde de 274 à 284 nm au spectre ultraviolet visible et par la présence de bandes d'une étendue pl de 4,2 à 8,4 sous focalisation isoélectrique au gel polyacrylamide à 5%, ledit complexe contenant un diluant pharmaceutiquement acceptable.

**2.** Composition pharmaceutique destinée à l'utilisation selon la revendication 1, dont le diluant est une solution tampon contenant des excipients et prête à l'emploi, comprenant des compositions d'une haute masse moléculaire, des stabilisateurs, des conservateurs et des agents de solubilisation.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010007620 A1
- WO 2009088314 A1
- RU 2128511
- RU 2104702
- RU 2049472

### Non-patent literature cited in the description

- Encyclopedia of Drugs. 2006, 970
- Guidelines for Experimental (Preclinical) Study of New Pharmacological Substances. OAO "Meditsina" Publishing House, 2005, 131-170
- Methodological Guidelines. Carcinogenicity Assessment of Pharmacological Drugs. Guidelines for Experimental (Preclinical) Study of New Pharmacological Substances. OAO "Meditsina" Publishing House, 2005, 100-122
- Methodological Guidelines. Carcinogenicity Assessment of Pharmacological Drugs and Excipients in Short-Term Tests. Guidelines for Experimental (Preclinical) Study of New Pharmacological Substances. OAO "Meditsina" Publishing House, 2005, 131-170
- Enhancement of Blood Supply to Ischemic Brain Under Effect of Afobazol. **I.V. SILKINA ; T.S. GAN'SHINA ; S.B. SEREDININ ; P.S. MIRZOYAN.** J. Exp. and Clin. Pharmacology. 2004, vol. 67, 9-13
- Models for studing, long-term recovery following forebrain ischemia in the rat. A 2-vessell occlusion model. **SMITH M.L. ; BENDEK G. ; DAHLGEN N.** Neurol.Scan. 1984, vol. 69, 385-400
- Φ **Y.WANG-FISHER.** Manual of Stroke Models in rats. CRC press, 2008, 3-4
- Laboratory Animals Care and Keeping Program. *Nursery for laboratory animals under Federal Institute of bioorganic chemistry,* November 2005